# EUROPEAN PATENT APPLICATION

(11) **EP 3 449 872 A1**
(43) Date of publication of application: **06.03.2019**
(21) Application number: 17188618.7
(22) Date of filing: 30.08.2017
(51) Int. Cl.: A61F 2/08

(54) **IMPLANT SYSTEM FOR TENDON-BONE INTERFACE REPAIR**

(71) Applicant: Universität Zürich, 8006 Zürich (CH)
(72) Inventor: SNEDEKER, Jess, 8008 Zürich (CH); BACHMANN, Elias, 8706 Meilen (CH); CAMENZIND, Roland, 8032 Zürich (CH); ZWYSSIG, Iwan, 6030 Ebikon (CH)
(74) Representative: Schulz Junghans Patentanwälte PartGmbB

(57) **Abstract**

The invention relates to an implant system (1) comprising an anchor (10) adapted to be inserted into a borehole (52) formed into a bone, particularly a humerus bone (50), and a flexible patch member (20) configured to be arranged in a groove (11) of said anchor (10) such that at least one end section (21,22) of the patch member (20) protrudes from the anchor (10), wherein said at least one end section (21,22) forms a fastening region (26) for connecting the respective end section (21,22) to a tendon or ligament, particularly a rotator cuff (60).

## Description

The invention relates to an implant system, particularly for rotator cuff repair in a shoulder of a human or animal. In particular, the implant system can also be used for repair of bone tendon or bone ligament interfaces in other joints, such as surgical repair of the Achilles tendon insertion to the calcaneus bone.

Clinical success in surgical repair of torn tendons and ligaments is often hindered by poor healing at the tendon-bone interface. Of the numerous clinical indications that would benefit from improved technologies and techniques to improve tendon to bone healing, the surgical repair of massive rotator cuff tendon tears represents a particularly urgent unmet clinical need. Tears of the rotator cuff are the most common cause for shoulder disability, with about 30 % to 50 % of the population older than 50 years being affected.

The rotator cuff consists of the supraspinatus, infraspinatus, subscapularis, and teres minor muscles and their associated tendons. The tendinous insertions of these four muscles form a cuff around the proximal humerus bone, whose main function is the stabilization of the humeral head in the glenoid cavity. The rotator cuff is frequently exposed to high loads and therefore susceptible to injury.

Rotator cuff tears can result from an acute traumatic injury, but more often from chronic wear over time induced by repetitive stresses. Repetitive arm motions for example during sport activities, can lead to chronic microtraumas resulting in a progressive degeneration of the rotator cuff tendons. Mechanical stresses often combine with age-related degenerative changes to finally cause a rotator cuff tear. Age is a major risk factor for the injury, since the severity of tendon matrix degeneration increases with time. Degenerative rotator cuff tears predominantly start in the supraspinatus tendon and further extend to adjacent structures.

The surgical technique for treating rotator cuff tears has evolved from a classic open approach to a mini-open and finally to an all arthroscopic approach. Suture anchors are commonly used to re-fixate the torn tendon to the humeral head. During arthroscopic repair, the sutures of the anchors are passed through the torn tendon and knots are subsequently tied to fix the tendon to the bone. Suture anchors are typically used to provide robust tendon to bone anchorage. Current suture anchors are available in different materials: metal (e.g. titanium), plastic (polyether ether ketone, PEEK), biodegradable (e.g. poly-l-lactide acid, PLLA) and biocomposites that include osteoconductive compounds (tricalcium phosphate, TCP, or hydroxyapatite).

In addition to the above-mentioned suture anchor repair devices, the rotator cuff tear can be augmented with a patch device. In this technique, a biologic or synthetic patch graft is integrated into the suture anchor repair to reinforce the weakened tendon. This technique is considered to be particularly useful in situations involving poor tissue quality with massive rotator cuff tears or in rotator cuff revision surgeries. Previous studies investigating the performance of patch augmentation rotator cuff repair have shown promising outcomes with improved initial biomechanical properties and increased patient satisfaction.

Despite the use of suture anchors and improved surgical techniques of the prior art, the re-tear rate of a repaired RC remains alarmingly high, with reported values ranging from 20 % to 94 %, depending on the study report. The common causes for repair failure are anchor pullout from the bone, suture material breakage, surgical knot loosening or suture pullout of the tendon.

The tendon-bone interface, also called the enthesis, is generally accepted to be the weakest point of a repaired rotator cuff. The healthy enthesis allows an effective stress transfer from tendon to bone and vice versa through its gradual change in structure and mechanical properties. Since the stiffness of tendons and bones are different, high stress levels tend to accumulate at the enthesis and make healing of the repaired rotator cuff more difficult.

Previous studies in animal models have shown reactive scar formation at the tendon-bone interface following rotator cuff repair surgeries. Consequently, the newly formed scar tissue is mechanically weaker compared to the previously intact enthesis. To conclude, the high re-tear rate after rotator cuff repair indicates inadequate and incomplete healing of the tendon-bone interface. An adequate reconstruction of the enthesis with restored physiological properties and sufficient primary stability is challenging for the surgeon, but required for tendon-bone healing and good clinical results for the patient.

Thus, the objective of the present invention is to provide an implant system for repair of rotator cuff tears which is improved in respect of the above-described disadvantages of the prior art.

This objective is attained by the subject matter of claim 1. Dependent claims 2 to 15 relate to favorable embodiments of the invention, which is described hereafter.

The invention relates to an implant system comprising an anchor (also referred to as patch anchor) adapted to be inserted into a borehole formed into a bone, particularly a humerus bone, more particularly a humeral upper extremity, most particularly a head or a tuberosity of the humeral upper extremity, and a flexible patch member configured to be arranged in a groove of the anchor such that a first end section of the patch member protrudes from the anchor and/or from the borehole when the anchor is inserted into the borehole, wherein the first end section forms a fastening region, particularly a first fastening region, for connecting the respective end section to tendon or ligament, particularly a rotator cuff, more particularly the supraspinatus, infraspinatus, teres minor and/or subscapularis tendons.

The borehole is particularly a blind-hole, in other words the borehole is not a through-hole, but comprises a bottom.

In addition, the at least one fastening region can be connected to at least one additional suture anchor (particularly, medial to the implant described herein) which is connected to, particularly inserted, in the bone, particularly the humerus bone.

Advantageously, the arrangement of the patch member in the groove of the anchor allows tight contact between the patch member and the bone, particularly the humerus bone. Therefore, when the patch member is connected to the tendon or ligament, particularly the rotator cuff, tendon and bone cells, and/or their progenitors, may be guided toward the enthesis: for instance, tendon cells may be guided toward the bone tunnel by means of the patch member, which improves healing of the tendon-bone-interface (enthesis). Similarly, skeletal progenitor cells, particularly within the humeral head, may be guided from the tunnel toward the tendon, whereby they assist in the regeneration of the enthesis.

In addition, the arrangement of the patch member in the groove of the anchor improves the mechanical connection between the patch member and the bone, particularly the humerus bone (by means of the anchor), which reduces the chance of detachment of the patch member from the bone, particularly the humerus bone after tendon or ligament, particularly rotator cuff, repair.

In certain embodiments, the patch member is configured to contact an inside wall and/or a bottom of the borehole when the patch member is arranged in the groove of the anchor and the anchor is inserted into the borehole.

This allows guiding of tendon/ progenitor cells to the bone, particularly the humerus bone in the borehole to restore the enthesis and further improves the stability of the mechanical connection between the patch member and the bone, particularly the humerus bone.

In certain embodiments, the patch member comprises a second end section opposite the first end section. In particular, the patch member extends between the first end section and the second end section along a patch axis when the patch member is arranged (for example stretched out) on a flat surface.

In certain embodiments, the implant system is configured such that the first end section and the second end section of the patch member protrude from the anchor and/or from the borehole when the patch member is arranged in the groove of the anchor and the anchor is inserted into the borehole, wherein the second end section forms a fastening region, particularly a second fastening region, for connecting the second end section to the tendon or ligament, particularly the rotator cuff, more particularly supraspinatus, infraspinatus, teres minor and/or subscapularis tendons. In other words, both end sections form a respective fastening region for connection of the respective end section to the tendon or ligament, particularly the rotator cuff.

This further improves the mechanical connection of the implant system to the tendon or ligament, particularly the rotator cuff, since two separate fastening regions are available, for example for connection to the tendons, particularly the rotator cuff tendons. In addition, one or both of the fastening regions can be connected to further anchors inserted medial to the implant in the bone, particularly the humerus bone.

In certain embodiments, the first end section comprises a width that is greater than a width of the second end section.

In particular, the width of the second end section is smaller than or equal to a breadth of the groove of the anchor, such that the second end section can be optimally arranged in the groove of the anchor.

In the scope of the present specification, the term 'width' describes an extension of the flexible patch member perpendicular to a patch axis, wherein the first and the second end sections extend along the patch axis when the patch member is stretched out on a flat surface. Of course, the patch member is not stretched out along a flat surface in the configuration in which the implant system is implanted in the bone, particularly the humerus bone and tendon or ligament, particularly rotator cuff. The flat surface is merely used to define the width of the patch member.

In particular, the first end section comprises a maximum width that is greater than a maximum width of the second end section.

The greater width of the first end section results in a larger surface area of the first end section, which is advantageous when the first end section is used as a fastening region for connection to the tendon or ligament, particularly the rotator cuff. Thereby, the mechanical connection between the patch member and the tendon or ligament, particularly the rotator cuff, is improved. In addition, the greater surface area of the first end section improves mechanical stress distribution to the repaired tendon or ligament, particularly the rotator cuff, and provides an enlarged surface for ingrowth of cells, particularly tendon cells.

Due to the smaller width of the second end section, the second end section can be arranged in the groove of the anchor more easily during insertion of the anchor along with the patch member into the borehole of the bone, particularly the humerus bone.

In certain embodiments, the first end section and the second end section of the patch member are connected to each other via a middle section of the patch member, wherein the middle section is configured to be arranged in the groove of the anchor, and wherein the middle section comprises a width that is smaller than a width of the first end section and smaller than a width of the second end section. In other words, the patch member can be described as having a butterfly shape.

In particular, the middle section comprises a maximum width that is smaller than a maximum width of the first end section and smaller than a maximum width of the second end section.

Due to the smaller width of the middle section compared to the end section, the middle section can be easily arranged in the groove of the anchor during insertion of the anchor along with the patch member into the borehole. The larger width of the two end sections serves to increase the surface area of the respective fastening regions, such that the mechanical connection of the fastening regions to the tendon or ligament, particularly the rotator cuff, and/or to medial anchors is improved. In addition, the greater surface area of the end sections improves mechanical stress distribution to the repaired tendon or ligament, particularly the rotator cuff, and provides an enlarged surface for ingrowth of cells, particularly tendon cells.

In certain embodiments, the anchor extends along a longitudinal axis, wherein the anchor is configured to be inserted into the borehole such that the longitudinal axis is arranged along the central axis of the borehole.

In particular, the anchor is shaped such that an envelope of the anchor is shaped as a cylinder, more particularly a cylinder having a circular base, wherein said longitudinal axis is a cylinder axis of the envelope.

In certain embodiments, the groove of the anchor extends parallel to the longitudinal axis, in other words the groove is arranged in an axial position with respect to the longitudinal axis.

This allows easy arrangement of the middle section or the second end section in the groove of the anchor. The orientation of the groove parallel to the longitudinal axis allows guiding of cell ingrowth deep into the borehole. Furthermore, due to the orientation of the groove, the first end section and/or the second end section of the patch member can be easily placed such that they protrude from the anchor and/or the borehole after insertion of the anchor into the borehole.

In certain embodiments, the groove comprises a breadth, in particular in a radial direction with respect to the longitudinal axis, that is perpendicular to the longitudinal axis, wherein the width of the middle section or the second end section of the patch member is smaller or equal to the breadth of the groove, such that the middle section or the second end section of the patch member can be arranged in the groove.

Alternatively, the middle section, the first end section and/or second end section can be deformed or compressed in order to be arranged in the groove. In particular, this is possible even if the width of the respective middle section or end section is greater than the breadth of the groove.

In certain embodiments, the implant system further comprises an insert, which is configured to be arranged in a recess of the anchor, in particular in a form fitting manner, such that the insert is configured to contact the bone, particularly the humerus bone, in particular an inside wall of said borehole, when the anchor is inserted into the borehole.

Therein, particularly, the insert serves as the osteoconductive or osteoinductive component to encourage biological fixation, also known as secondary fixation. For example, the insert is mechanically connected to the anchor by means of bone tissue ingrowth.

By means of a separate secondary insert, a smaller osteoconductive or osteoinductive component can be used, such that the remaining force transmitting component for primary fixation can be manufactured from a cheaper or more easily producible material. This advantageously reduces the cost of the implant system according to the invention.

In addition, the insert may be configured to improve the stability of the anchor in the borehole, particularly against pullout from the borehole.

In certain embodiments, the anchor comprises a first side, a second side opposite the first side, and a bottom side connecting the first side and the second side, wherein the groove extends along the first side, the bottom side and the second side.

In particular, the first side and the second side are arranged in a circumferential direction with respect to the longitudinal axis. More particularly, the first side and the second side are arranged in an angle of 180° with respect to each other around a circumference of a cylinder-shaped envelope of the anchor, wherein the cylinder axis of the envelope is parallel to the longitudinal axis of the anchor.

In particular, the bottom side is arranged parallel to an end face of the cylinder-shaped envelope of the anchor.

When the anchor is inserted into the borehole in the bone, particularly the humerus bone, the first side and the second side face an inside wall of the borehole, and the bottom side faces the bottom of the borehole.

In certain embodiments, the anchor comprises a top side connecting the first side and the second side, wherein the recess of the anchor is open towards the first side, the second side and the top side.

In particular, the top side is arranged parallel to an end face of the cylinder-shaped envelope of the anchor.

When the anchor is inserted into the borehole, the top side of the anchor faces the top of the borehole and is accessible from the outside. Therefore, when the recess is open towards the top side, the insert can be conveniently inserted into the recess after insertion of the anchor into the borehole.

In particular, in case the recess is open to the first and second side, the insert contacts the patch member in the groove of the anchor, and indirectly contacts the bone, particularly the humerus bone, via the patch member when the anchor is inserted into the borehole, the patch member is arranged in the groove of the anchor, and the insert is arranged in the recess of the anchor.

In certain embodiments, the anchor comprises a third side connecting the first side and the second side, and a fourth side opposite the third side, wherein the recess is open towards third side, the fourth side and the top side.

Therein, in particular, the groove and the recess are spaced apart from each other on the anchor, in other words the groove and the recess do not overlap.

Thereby, both the patch member arranged in the groove and the insert arranged in the recess are allowed to directly contact the bone, particularly the humerus bone (specifically the inside walls and/or the bottom of the borehole), which improves ingrowth of tendon/ bone cells and healing of the enthesis.

In particular, the third side and the fourth side are arranged in a circumferential direction with respect to the longitudinal axis.

More particularly, the third side is arranged between the first side and the second side around the circumference of the cylinder-shaped envelope of the anchor, most particularly in an angle of 90° with respect to the first side and the second side.

In particular, the fourth side is arranged between the first side and the second side around the circumference of the cylinder-shaped envelope of the anchor, more particularly in an angle of 90° with respect to the first side and the second side.

Therein, the third side and the fourth are arranged opposite to each other, particularly in an angle of 180° with respect to each other around the circumference of the cylinder-shaped envelope of the anchor.

When the anchor is inserted into the borehole, the third side and the fourth side face an inside wall of the borehole.

In certain embodiments, the anchor comprises or consists of an osteoinductive or osteoconductive material. This further improves restoration of the tendon-bone interface after tendon or ligament, particularly rotator cuff, repair.

Therein the term 'osteoinductive' describes a material which is adapted to serve as a scaffold for new bone growth that is perpetuated by the native bone. The term 'osteoconductive' describes a material which is adapted to stimulate osteoprogenitor cells to differentiate into osteoblast, thereby facilitating new bone formation.

In certain embodiments, the anchor comprises an osteoconductive and/or osteoinductive component and a force transmitting component. In other words, the anchor comprises at least a first part or component and a second part or component, which may be mechanically connected, wherein the first part comprises an osteoconductive or osteoinductive material, and the second part is configured to provide mechanical stability and/or rigidity to the anchor.

In particular, the osteoinductive osteoconductive component comprises or consists of tricalcium phosphate, hydroxyapatite, calcium phosphate, calcium silicate and/or human or animal bone.

In other words, in case bone is used as osteoinductive or osteoconductive component, the component may be categorized as xenograft or allograft.

Therein the term xenograft refers to a transplant from a donor which belongs to a different species than the recipient of the transplant, for example a bovine bone implanted in a human recipient.

The term allograft refers to a transplant from a donor which is non-identical, but from the same species as the recipient of the transplant, for example a bone of a first human implanted into a second human.

In particular, the force transmitting component comprises or consists of polylactic acid (PLA), polylactic-co-glycolic acid (PLGA), poly-ε-caprolactone (PCL), polyhydroxyalkanoate (PHA), polyether ether ketone (PEEK), titanium, a titanium alloy and/or bone, more particularly bovine bone.

In certain embodiments, the anchor comprises or consists of tricalcium phosphate, hydroxyapatite, calcium phosphate, calcium silicate and/or human or animal bone.

In certain embodiments, the anchor comprises or consists of polylactic acid (PLA), polylactic-co-glycolic acid (PLGA), poly-ε-caprolactone (PCL), polyhydroxyalkanoate (PHA), polyether ether ketone (PEEK), titanium, a titanium alloy and/or bone, more particularly bovine bone.

In certain embodiments, the insert comprises or consists of an osteoinductive or osteoconductive material.

In certain embodiments, the osteoinductive or osteoconductive material is selected from the group consisting of tricalcium phosphate, hydroxyapatite, calcium phosphate, calcium silicate and/or human or animal bone.

In certain embodiments, the patch member comprises a first layer (also referred to as 'biological augmentation layer') comprising a biocompatible material, particularly collagen, and a second layer connected to the first layer. Therein, the first layer and the second layer may be sandwich-like superimposed or woven into a single interconnecting layer.

Advantageously, the biocompatible material of the first layer improves attachment of cells, particularly tendon/ bone cells, and/or their progenitors, to the patch member and thereby facilitates ingrowth of the cells in the tendon-bone interface or enthesis. The patch also advantageously supports ingrowth and appropriate migration of cells. These aspects improve healing of the enthesis after tendon or ligament, particularly rotator cuff, repair. Additionally, the second layer may provide mechanical stability to the patch member, such that rupture of the patch member upon mechanical stress is avoided, and/or may be used to release compounds into the tissue surrounding the implant system.

In particular, the collagen is a spongy collagen and/or is in a sheet form of aligned fibers.

In particular, the second layer comprises or consists of a textile, more particularly a braided textile.

In certain embodiments, the patch member, particularly the second layer, comprises a tear strength of at least 200 N when fully hydrated.

In the scope of the present specification, the term 'fully hydrated' refers to a state of the patch member after the patch member is placed in water or an aqueous solution (for example a buffer solution) for at least 5 minutes.

In the scope of the present specification, the term 'tear strength' refers to the maximum force which can be applied along a length of the patch member extending along a patch axis from the first end section to a second end section opposite the first end section of the patch member before tearing of the patch member or second layer.

In certain embodiments, the patch member, particularly the second layer, comprises an axial stiffness of at least 20 N/mm when fully hydrated.

In the scope of the present specification, the axial stiffness is defined by the formula a=(E*A)/I, wherein a is the axial stiffness, E is the Young's modulus of the patch member, I is a length of the patch member extending along a patch axis from the first end section to a second end section opposite the first end section of the patch member, and A is the cross-sectional area of the patch member perpendicular to the length I.

In other words, the second layer is configured to increase the strength of the patch member when immersed in body fluids. Therein, the term strength refers to mechanical stability, in particular against rupture.

A strong, mechanically stable, patch member is advantageous both during implantation surgery and after implantation.

In certain embodiments, the patch member, particularly the second layer, comprises a longitudinal bending stiffness of at least 0,0005 Nm, and a lateral bending stiffness of at least 0,005 Nm, when fully hydrated.

In the scope of the present specification, bending stiffness (Taber test of flexural rigidity, ISO 2493-2:2011), is defined as the bending moment (measured in Nm) required to deflect the free end of a 38 millimeter wide sample of the patch member material or second layer material, which is vertically clamped at a clamp interface, 15 degrees from its center line, when an orthogonal load is applied 50 millimeters away from the clamp interface, wherein the orthogonal load is applied perpendicular to a plane of extension of the patch member or second layer.

The term 'longitudinal bending stiffness' refers to the bending stiffness (according to the above-described Taber test) when the sample is clamped such that a straight line between the clamp interface and the point at which the orthogonal load is applied to the sample is parallel to the patch axis.

The term 'lateral bending stiffness' refers to the bending stiffness (according to the above-described Taber test) when the sample is clamped such that a straight line between the clamp interface and the point at which the orthogonal load is applied to the sample is perpendicular to the patch axis.

In certain embodiments, the patch member and/or the second layer comprises at least one reinforcing structure for increasing the lateral bending stiffness of the patch member and/or second layer. For example, such a reinforcing structure may be a strut incorporated into the patch member and/or second layer material, wherein the strut is perpendicular to the patch axis.

In other words, the patch member and/or the second layer comprises an anisotropic stiffness, in particular deriving from its material properties and/or from reinforcing structures.

In particular, the patch member and/or second layer is pliant along the patch axis, such that it may fold along the patch axis. This is advantageous for the surgical technique during implantation of the implant system, in particular since folding of the patch member along the groove of the anchor is facilitated.

A higher lateral bending stiffness facilitates handling of the patch member, in particular during implantation, because the shape of the patch member is retained and lateral folding onto itself during handling is prevented.

In particular, the second layer is configured to increase the shape-holding properties of the patch member. Therein, the term "shape-holding properties" refers to the ability of the patch member to hold its shape or return to its original shape, in particular its lateral dimensions, and further when the patch member is deformed by external forces in a fluid environment.

In certain embodiments, the patch member, particularly the second layer, comprises and/or is configured to release a biologically active compound, a drug, and/or a cross-linking agent, wherein the biologically active compound, the drug, and/or the cross-linking agent is adapted to diffuse into tissue surrounding the patch member when the patch member is arranged in the groove of the anchor and the anchor is inserted into the borehole.

In certain embodiments, the patch member, particularly the second layer, comprises a delivery system for controlled release of a biologically active compound, a drug, and/or a cross-linking agent into tissue surrounding the patch member when the patch member is arranged in the groove of the anchor and the anchor is inserted into the borehole.

In particular, the biologically active compound is platelet rich plasma, a recombinant or a natural growth factor, a small molecule peptide which is adapted to inhibit or stimulate a resident cell behavior, an anti-inflammatory agent, or a small molecule pharmaceutical agent which is adapted to stimulate mineralized and/or non-mineralized collagen production by cells, particularly cells at the enthesis.

In the scope of the present specification, the term 'small molecule' refers to a molecule having a molecular weight of less than 900 Da.

In certain embodiments, the cross-linking agent is a collagen cross-linking agent which is adapted to cross-link collagen.

In certain embodiments, the cross-linking agent is genipin (CAS-No. 6902-77-8).

In particular, such a cross-linking agent enables biomechanical augmentation of the overall construct, in other words the patch member and/or the implant system, in particular chemically cross-linking the local tissue (for example the tendons of the rotator cuff) around the implant system, particularly around the patch member.

In certain embodiments, the implant system comprises at least one medial anchor in addition to the anchor for insertion into the borehole, wherein the at least one medial anchor connecting the patch member to the bone, particularly the humerus bone, wherein the at least one medial anchor is configured to be inserted into the bone, particularly the humerus bone.

Such additional medial anchors additionally improve the stability and resistance against mechanical stress of the implant system.

The invention is further illustrated by the following examples and Figures, from which additional embodiments may be derived. Therein
- Fig. 1: shows a schematic perspective view of an implant system according to the present invention,
- Fig. 2: shows a schematic perspective view of a first embodiment of an anchor and insert according to the invention,
- Fig. 3: shows a schematic perspective view of a second embodiment of an anchor and insert according to the invention,
- Fig. 4: shows schematic views of different embodiments of a patch member according to the invention,
- Fig. 5: is a schematic representation of a rotator cuff repair, wherein an implant system according to the invention is inserted into a humerus bone and attached to a torn rotator cuff,
- Fig. 6: shows load to failure curves of a patch member according to the invention compared to collagen strips of the prior art,
- Fig. 7: shows prototypes of components of the implant system according to the invention,
- Fig. 8: shows results of Ultimate Tensile Strength measurements performed on anchors according to the invention compared to suture anchors of the prior art,
- Fig. 9: shows results of cyclic loading tests performed on anchors according to the invention compared to suture anchors of the prior art,
- Fig. 10: shows an arthroscopic surgery on a sheep shoulder using the implant system according to the invention,
- Fig. 11: shows an open surgery on a sheep shoulder using the implant system according to the invention.

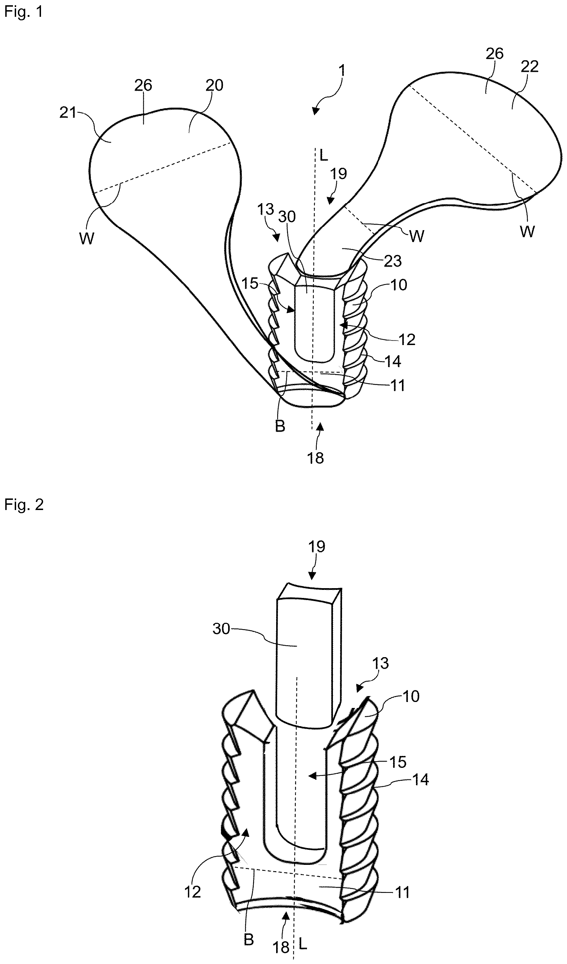

Fig. 1 shows an implant system 1 according to the invention comprising an anchor 10 for insertion into a borehole 52 of a humerus bone 50, a flexible patch member 20 configured to form at least one fastening region 26 for connecting the patch member 20 to a rotator cuff 60, and an osteoconductive insert 30 for contacting the humerus bone 50.

The anchor 10 extends along a longitudinal axis L and comprises a first side 12 and a second side 13 positioned in a circumferential direction with respect to the longitudinal axis L. Furthermore, the anchor 10 comprises a bottom side 18 and a top side 19, wherein the bottom side 18 and the top side 19 are arranged along the longitudinal axis L.

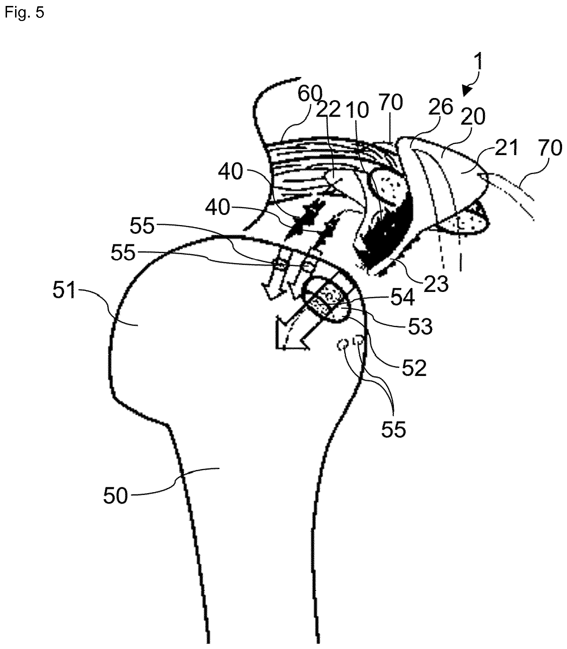

When the anchor 10 is inserted into a borehole 52 of a humerus bone 50 (see Fig. 5), the first side 12 and the second side 13 face an inside wall 53 of the borehole 52, the bottom side 18 faces a bottom 54 of the borehole 52, and the top side 19 faces the opening of the borehole 52.

A groove 11 for insertion of the patch member 20 extends along the first side 12, the second side 13 and the bottom side 18 of the anchor 10, wherein the part of the groove 11 extending along the first side 12 and the second side 13 is parallel to the longitudinal axis L, in other words extending along an axial direction in respect of the longitudinal axis L.

Furthermore, the anchor 10 comprises a recess 15, in which the insert 30 is placed. The recess 15 is open towards the first side 12 and the second side 13 in the radial direction in respect of the longitudinal axis L (perpendicular to the longitudinal axis L), such that the insert 30 may indirectly contact the humerus bone 50 surrounding the anchor 10 via the patch member 20 when the anchor 10 is inserted into a borehole 52 in the humerus bone 50, thereby improving tenocyte ingrowth into the humerus bone 50.

The anchor 10 further comprises notches 14 which are configured to improve fixing of the anchor 10 in the borehole 52 of the humerus bone 50, thereby preventing pullout of the anchor 10 from the borehole 52. In the example shown in Fig. 1 the notches 14 are arranged in a circumferential direction with respect to the longitudinal axis L, thereby improving static friction between the anchor 10 and the inside walls 53 of the borehole 52.

The flexible patch member 20 comprises end sections 21,22 which are connected via a middle section 23, wherein a width W, in particular a maximum width W, of the middle section 23 is smaller than the width W of the end sections 21,22. In the example depicted in Figure 1, the width W of the middle section 23 is smaller or equal to a breadth B of the groove 11 of the anchor 10 in a radial direction with respect to the longitudinal axis L. In this manner, the middle section 23 of the patch member 20 can be easily placed into the groove 11 during insertion of the anchor 10 into the borehole 52.

When the anchor 10 is inserted into the borehole 52, the end sections 21,22 protrude from the anchor 10 and form fastening regions 26 for connecting the patch member 20 to a rotator cuff 60.

Fig. 2 shows a schematic perspective view of a first embodiment of an anchor 10 for insertion into a borehole 52 of the humerus bone 50 and an osteoconductive insert 30, constituting components of an implant system 1 according to the invention.

The anchor 10 extends along a longitudinal axis L. On the opposite first side 12 and second side 13 (180° to each other around the circumference with respect to the longitudinal axis), and the bottom side 18 of the anchor 10, a groove 11 is formed wherein the groove 11 has a breadth B in a radial direction with respect to the longitudinal axis L.

Furthermore, the anchor 10 comprises a recess 15 for insertion of the insert 30, which in the embodiment shown in Fig. 2 is open towards the first side 12, the second side 13 and the top side 19.

The groove 11 is adapted such that a section of the patch member 20, in particular the middle section 23, can be arranged in the groove 11 when the anchor 10 is inserted in a borehole 52 of the humerus bone 50.

As previously described for the anchor 10 shown in Fig. 1, the anchor 10 comprises notches 14 for fixing the anchor 10 in a borehole 52 of the humerus bone 50, which are arranged in a circumferential direction with respect to the longitudinal axis L on the anchor 10.

The insert 30 is adapted to be inserted and tightly fixed in the recess 15 of the anchor 10. Thereby, the insert 30 improves fixing of the anchor 10 in a borehole 52 of the humerus bone 50 and in particular the resistance of the anchor 10 against pullout from the borehole 52 during operation of the repaired rotator cuff 60.

In addition, since the recess 15 is open in a radial direction in respect of the longitudinal axis L (towards the first side 12 and the second side 13 in this embodiment), the insert 30 may contact the humerus bone 50 directly or indirectly via the patch member 20 when the anchor 10 is placed in the borehole 52 of the humerus bone 50. Therefore, in particular due to its osteoconductive and/or osteoinductive properties, the insert 30 can improve healing of the tendon to bone interface (enthesis) by directly or indirectly contacting the humerus bone 50.

In particular, the insert 30 may consist of or comprise any osteoconductive and/or osteoinductive material, for example tricalcium phosphate, hydroxyapatite, calcium phosphate, calcium silicate and/or human or animal bone.

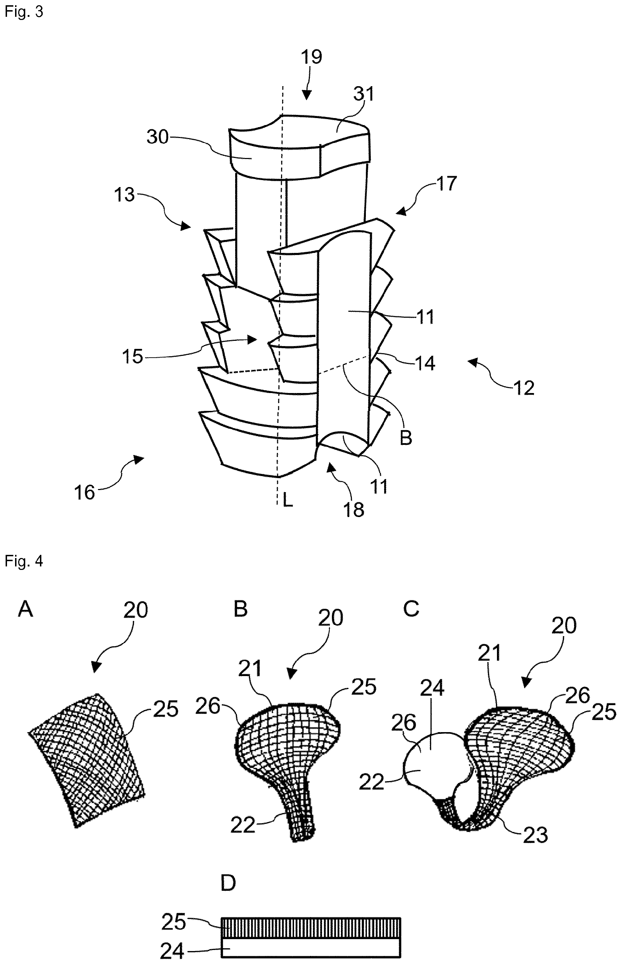

Fig. 3 shows a schematic perspective view of a second embodiment of an anchor 10 for insertion into a borehole 52 of the humerus bone 50 and an osteoconductive insert 30, constituting components of an implant system 1 according to the invention.

Similar to the anchor 10 depicted in Fig. 2, the anchor 10 shown in Fig. 3 extends along a longitudinal axis L. The anchor 10 also comprises a groove 11 along its first side 12, its second side 13 and its bottom side 18. The anchor 10 further comprises a recess 15 for insertion of an insert 30. However, in contrast to the embodiment of Fig. 2, the recess 15 is open towards a third side 16 and a fourth side 17. In other words, the groove 11 and the recess 15 are arranged perpendicular to each other when viewed in a cross-section with respect to the longitudinal axis L, and the recess 15 is positioned separately from the groove 90° apart around the circumference of the anchor 10.

This results in a better direct contact of the insert 30 to the humerus bone 50 when the anchor 10 is placed in the borehole 52 and the insert 30 is placed in the recess 15, since the patch member 20 does not obstruct the recess 15 when the patch member 20 is arranged in the groove 11.

The insert 30 is adapted to be fixed inside the recess 15, such that the insert 30 improves fixing of the anchor 10 in the borehole 52 and resistance of the anchor 10 against pullout from the borehole 52.

In particular, similar to the insert 30 shown in Fig. 2, the insert 30 may consist of or comprise any osteoconductive and/or osteoinductive material, such as tricalcium phosphate, hydroxyapatite, calcium phosphate, calcium silicate, and/or human or animal bone.

Fig. 4 shows different embodiments of flexible patch members 20 for connecting a rotator cuff 60 to the implant system 1 according to the invention.

The patch member 20 shown in Fig. 4A has a rectangular shape and can be cut into a desired shape prior to insertion of the patch member 20 into the groove 11 of the anchor 10 and insertion of the anchor 10 into a borehole 52 of the humerus bone 50.

Fig. 4B depicts a patch member 20 with a first end section 21 forming a fastening region 26 for connecting the first end section 21 to a rotator cuff 60, and a second end section 22 for insertion into the groove 11 of the anchor 10. The first end section 21 comprises a greater width W (see Fig. 1) than the second end section 22. In turn, the first end section 21 comprises a relatively large surface area, such that it can be easily and tightly connected to the rotator cuff 60, whereas the second end section 22 is sufficiently narrow to fit into the groove 11 of the anchor 10, such that the anchor 10 can be easily inserted into the borehole 52 along with the patch member 20 and is held in place by the groove 11.

Furthermore, the width W of the second end section 22 is smaller than the breadth B of the groove 11 of the anchor 10, such that the second end section 22 can be easily inserted into the groove 11.

When the anchor 10 is inserted into the borehole 52 along with the second end section 22 of the patch member 20 according to Fig. 4B, the first end section 21 protrudes from the anchor 10 and the borehole 52, such that the first end section 21 can be used as a fastening region 26 for connection of the patch member 20 to the rotator cuff 60, for example by means of sutures 70.

In particular, the second end section 22 of the patch member 20 may be fixed to the anchor 10 by additional fixing means to hold the second end section 22 in place, in particular when a pulling force acts on the patch member during normal operation of a repaired rotator cuff 60.

Fig. 4C shows a patch member 20 comprising a first end section 21 and a second end section 22, which are both adapted to serve as a fastening region 26 for connecting the patch member 20 to a rotator cuff 60, wherein the first end section 21 and the second end section 22 are connected by a middle section 23 for insertion into the groove 11 of the anchor 10.

The first end section 21 and the second end section 22 both comprise a width W (see Fig. 1) which is greater than the width W of the middle section 23, such that the surface area of the first and second end section 21,22 is improved for better fixing to the rotator cuff 60.

Furthermore, the width W of the middle section 23 is smaller the breadth B of the groove 11 of the anchor 10, such that the middle section 23 can be easily inserted into the groove 11.

When the anchor 10 is inserted into the borehole 52 along with the patch member 20, the first end section 21 and the second end section 22 protrude from the borehole 52, such that the end sections 21,22 can be used as fastening regions 26 for connection of the patch member 20 to the rotator cuff 60.

In addition, the middle section 23 which is inserted into the groove 11 in the anchor 10 placed in the borehole 52 holds the first end section 21 and the second end section 22 tightly in place and protects them against pullout from the borehole 52.

Fig. 4D shows a cross-sectional view of a patch member 20, which may comprise any suitable shape, in particular a shape according to any of the embodiments shown in Fig. 4A to 4C.

The patch member 20 comprises a first layer 24 comprising a biocompatible material, particularly collagen, more particularly a spongy collagen, and a second layer 25 connected to the first layer, wherein the second layer 25 is configured to increase the rigidity of the patch member 20.

For example, the second layer 25 may comprise or consist of polyethyleneterephthalat (PET), ultra-high molecular weight polyethylene (UHMWPE), silk, or absorbable material such as polylactic acid (PLA), poly (lactic-co-glycolic acid) (PLGA), or poly-e-caprolactone (PCL).

In addition, the second layer 25 may be loaded with platelet rich plasma, or may contain pharmaceutical compounds with anti-inflammatory properties, or other bioactive agents for controlled release, and/or cross-linking agents such as genipin for controlled release.

The second layer 25 improves the mechanical rigidity of the patch member 20 and therefore prevents rupture of the patch member 20 during or after rotator cuff 60 repair.

Without wishing to be bound by theory, the biocompatible first layer 24 allows attachment of tendon or bone cells, or their progenitors (enthesis-forming cells), which improves healing of the enthesis after rotator cuff 60 repair by promoting ingrowth of tendon/ bone cells into the borehole 52 in the humerus bone 50.

All embodiments of the patch member 20 according to the invention, in particular those depicted in Fig. 1, Fig. 4 and Fig. 5, may be freely combined with any of the embodiments of the anchor 10 and insert 30 according to the invention, in particular those shown in Fig. 1 to Fig. 3 and Fig. 5.

Fig. 5 schematically shows the insertion of an implant system 1 according to the invention into a human or animal humerus bone 50, and attachment of the implant system 1 to a torn rotator cuff 60 in order to repair the rotator cuff 60.

Therein, a borehole 52 comprising inside walls 53 and a bottom 54, is introduced into a humeral head 51 of a humerus bone 50. Subsequently, an anchor 10 comprising a groove 11 (see Fig.1 to Fig. 3) in which a middle section 23 of a patch member 20 (see Fig. 4C) is placed, is inserted into the borehole 52. The implant system 1 may or may not further comprise an insert 30 which is inserted into the recess 15 of the anchor 10 (see Fig. 2 and Fig. 3).

After the anchor 10 along with the patch member 20 has been inserted into the borehole 52, the first end section 21 and the second end section 22 protrude from the anchor 10 and the borehole 52 and are connected to the rotator cuff 60, in particular to tendons attached to muscles of the rotator cuff 60, by means of sutures 70.

Therein, the large surface area of the end sections 21,22 of the patch member due to their width W (see Fig. 1) allows especially easy placement of sutures 70 and improves the stability of the connection to the rotator cuff 60. The narrower middle section 23 of the patch member 20 allows easy insertion of the patch member 20 into the groove 11 of the anchor 10 and improves fixing of the anchor 10 and the patch member 20 in the borehole 52.

Due to the biocompatibility of the first layer 24 of the patch member 20 (see Fig. 4), tendon cells can attach to the first layer 24 and grow into the borehole 52, guided by the patch member 20 which is inserted into the borehole 52 by its middle section 23. Consequently, restoration of the bone-tendon interface (enthesis) after rotator cuff 60 repair is improved by the ingrowth of the tendon/ bone cells into the borehole 52. This in turn reduces the chance of recurring rotator cuff 60 injury.

The second layer 25 improves the mechanical rigidity of the patch member 20 and therefore reduces the chance of rupture of the patch member 20 during or after rotator cuff 60 repair.

In addition to the anchor 10, medial anchors 40 can be inserted at medial anchor positions 55 in the humeral head 51 and connected to the fastening regions 26 provided by the first end section 21 and second end section 22 of the patch member 20 by means of sutures 70. This further improves fixing of the implant system 1 on the humerus bone 50 and thus the stability of the repaired rotator cuff 60.

Different tests, which were performed to show the different functional parts of the implant system, are described hereafter.

### Example 1 - Mechanical properties of the patch member

A collagen matrix patch (Mucograft®, Geistlich 20x30mm) was cut into 3 stripes (6,5x30mm) and tested with a uniaxial material testing machine (Zwick 010, Zwick & Roell). Since collagen patches behave differently depending on being dry or wet, three experiments were performed. Sample one was kept dry, sample two was soaked with phosphate buffered saline (PBS) solution and sample three was reinforced with a layer of braided sutures (UHMWPE, Meister & Cie AG).

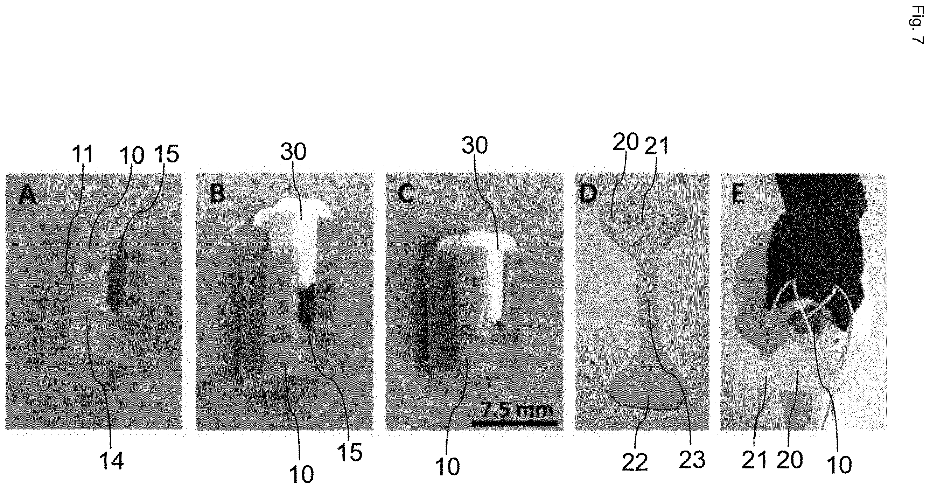

Fig. 6 shows load to failure curves of a collagen strip in dry condition 81, a collagen strip in wet condition 82, and a patch member according to the invention (mechanically augmented collagen strip) in wet condition 83.

As displayed in Fig. 6, a mechanical augmentation of the collagen layer (first layer) with a braided textile layer (second layer) increases pullout resistance of more than 400 % when compared to a single collagen layer.

### Example 2 - Initial stability of the anchor

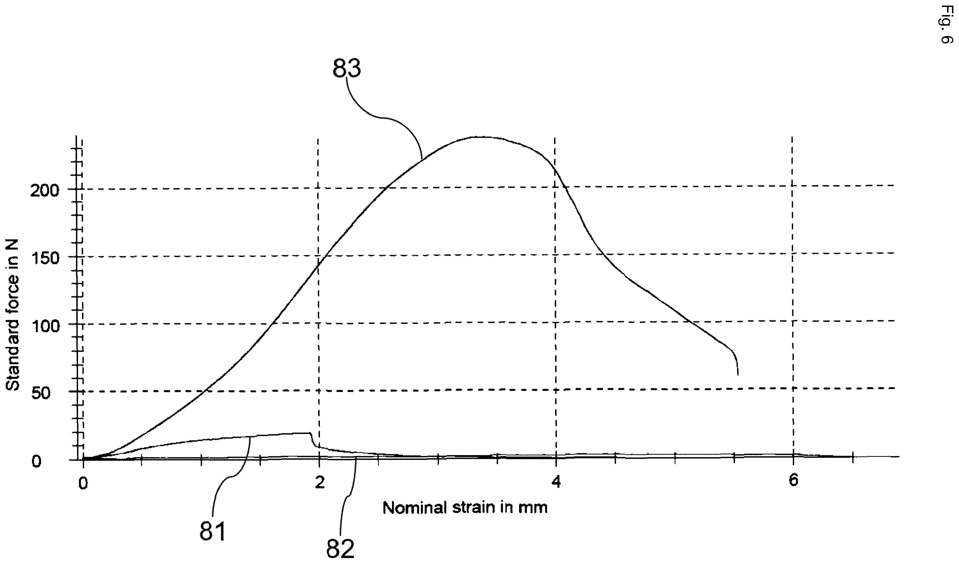

Anchor 10 and insert 30 prototypes (Fig. 7A to 7C) were fabricated and tested. Furthermore, conceptual prototypes of the patch member 20 and the implant system 1 (Fig. 7D to 7E) were developed for visualization reasons. Uniaxial pullout tests were performed with the anchor 10 prototypes to compare the anchor 10 according to the invention with suture anchors according to the prior art.

Two identical designs of different sized anchor 10 prototypes were fabricated (6,5 mm and 7,5 mm diameter) for comparison tests (in the following also referred to as 'Patch anchor 6.5' and 'Patch anchor 7.5').

The anchor 10 consisted of PEEK and the insert 30 was imitated with a 3D-printed polylactic acid material (PLA) (Fig. 7B to 7C). A 40/20 PFC sawbone foam block and porcine femur models served as test models. The drill hole for the 6,5-mm anchor 10 had a depth of 11 mm and the drill hole for the 7,5-mm anchor 10 had a depth of 13 mm. The boreholes were drilled 0,5 mm smaller in diameter than the anchor's outer diameter. To imitate the patch member, the anchors 10 were loaded with two 0,54 mm cable wire loops.

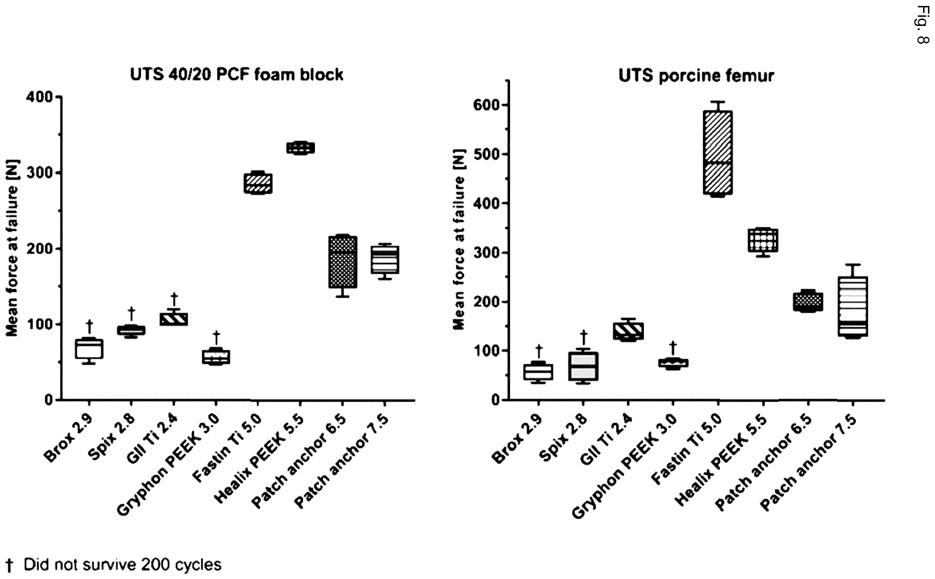

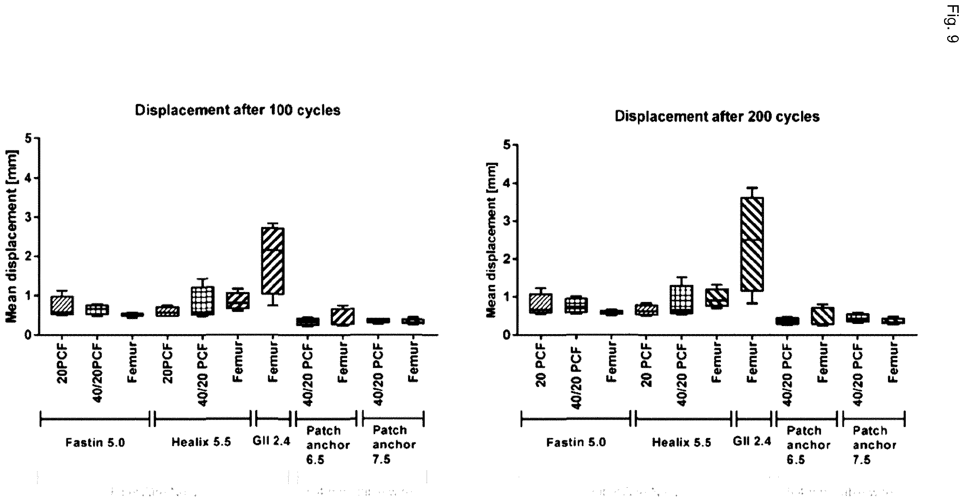

The anchor 10 was inserted using a flat, rectangular insertion tool with the size of the recess 15 of the anchor 10. The insert 30 was then pushed-in by hand and finally hammered in with a pin. Ultimate Tensile Strength (UTS) tests (Fig. 8) and cyclic loading tests (Fig. 9) were performed on a universal material testing machine (Zwick Z010, Zwick GmbH, Ulm, Germany) mounted with a 10 kN load cell (Zwick Xforce P, Zwick GmbH, Ulm, Germany) and compared to suture anchors of the prior art (Healix PEEK 5,5 mm anchor, Fastin Titanium 5,0 mm anchor, Gryphon PEEK 3,0 mm anchor and GII Titanium 2,4 mm anchor; DePuy Mitek, Johnson & Johnson, New Brunswick, NJ, USA).

The anchor 10 according to the invention showed promising Ultimate Tensile Strength results in both diameters (Fig. 8). Furthermore, the displacement of the anchor 10 was lower compared to all previously tested suture anchors (Fig. 9). However, sutures were not the same in these tests (0,54 mm cable wire vs. FiberWire No. 2) and the anchor 10 diameters (7,5 mm and 6,5 mm) were larger than those of the compared suture anchors. Regarding the performance of the 6,5 mm anchor 10 and the 7,5 mm anchor 10, no difference was seen. In conclusion, the anchor 10 performance is, according to the generated results adequate and the anchor 10 should remain stable after implantation. Moreover, the 6,5 mm anchor 10 size is adequate and might allow for an even smaller diameter.

### Example 3 - Feasibility of surgical technique

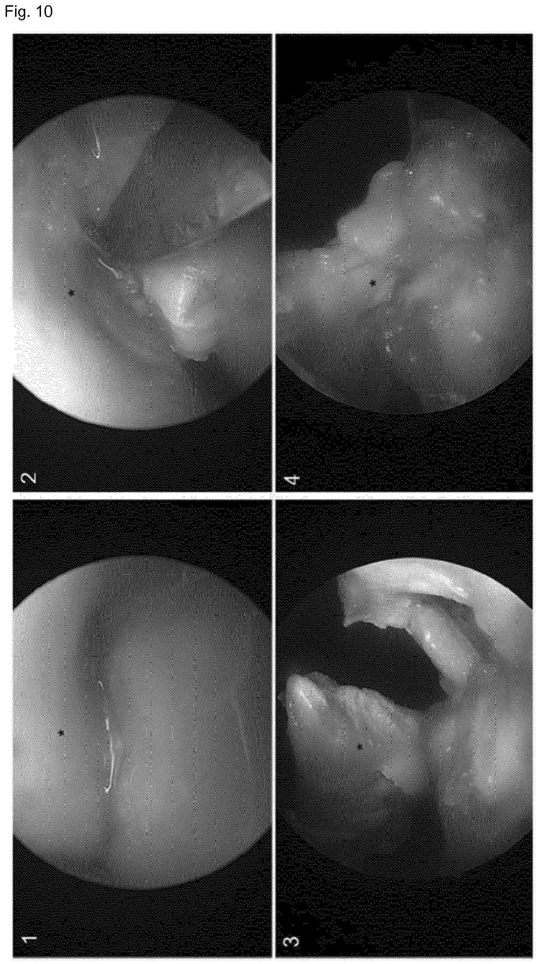

The feasibility of an arthroscopic pilot study of the tendon repair technique was tested on a sheep shoulder (Fig. 10). Therein, frame (1) shows an arthroscopic view of the intact ovine infraspinatus tendon (marked with *); frame (2) shows the disinsertion of the infraspinatus tendon (*) at the tendon-bone interface from intraarticular with an arthroscopic knife; frame (3) shows the medial part of the displaced infraspinatus tendon (*); frame (4) shos an intraarticular view of the repaired infraspinatus tendon (*).

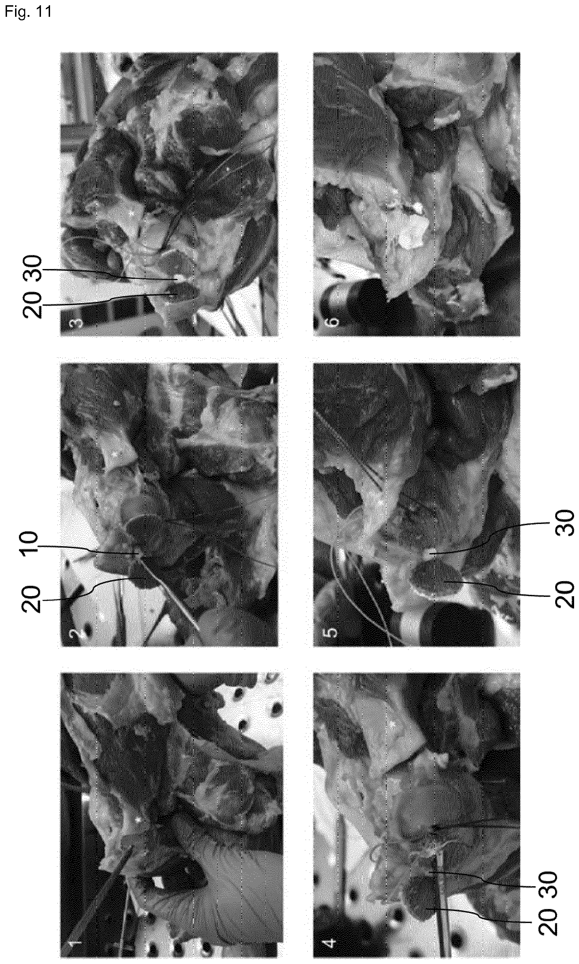

For a better explanation of the surgical technique, Fig. 11 shows the different steps in an open ovine shoulder joint. Therein, frame (1) shows insertion of a 5.0 Fastin Ti anchor at the footprint of the humeral head; frame (2) shows insertion of the implant system 1 comprising an anchor 10 (6,5 mm patch anchor) and a patch member 20 into a 6,0 mm drilled borehole in the humerus bone; frame (3) shows the situation after insertion of an insert 30 (also termed TCP device) into the anchor 10; frame (4) shows retrieving of a suture through the patch member 20 with a suture pass device; frame (5) shows retrieving of the suture through the infraspinatus tendon with a suture pass device; frame (6) shows the repaired infraspinatus tendon.

**List of reference signs**

| | |
|---|---|
| Implant system | 1 |
| Anchor | 10 |
| Groove | 11 |
| First side | 12 |
| Second side | 13 |
| Notch | 14 |
| Recess | 15 |
| Third side | 16 |
| Fourth side | 17 |
| Bottom side | 18 |
| Topside | 19 |
| Patch member | 20 |
| First end section | 21 |
| Second end section | 22 |
| Middle section | 23 |
| First layer | 24 |
| Second layer | 25 |
| Fastening region | 26 |
| Insert | 30 |
| Head | 31 |
| Medial anchor | 40 |
| Bone, particularly humerus bone | 50 |
| Humeral head | 51 |
| Borehole | 52 |
| Inside wall | 53 |
| Bottom | 54 |
| Medial anchor position | 55 |
| Tendon or ligament, particularly rotator cuff | 60 |
| Suture | 70 |
| Collagen strip in dry condition | 81 |
| Collagen strip in wet condition | 82 |
| Patch member in wet condition | 83 |
| Longitudinal axis | L |
| Breadth | B |
| Width | W |

## Claims

1. Implant system (1) comprising:
- an anchor (10) adapted to be inserted into a borehole (52) formed into a bone (50), and
- a flexible patch member (20) configured to be arranged in a groove (11) of said anchor (10) such that a first end section (21) of the patch member (20) protrudes from the anchor (10), wherein said first end section (21) forms a fastening region (26) for connecting the first end section (21) to a tendon or ligament (60).

2. Implant system (1) according to claim 1, **characterized in that** said patch member (20) is configured to contact an inside wall (53) and/or a bottom (54) of said borehole (52) when the patch member (20) is arranged in the groove (11) of the anchor (10) and the anchor (10) is inserted into the borehole (52).

3. Implant system (1) according to claim 1 or 2, **characterized in that** said patch member (20) comprises a second end section (22) opposite the first end section (21).

4. Implant system (1) according to claim 3, **characterized in that** said implant system (1) is configured such that the first end section (21) and the second end section (22) of the patch member (20) protrude from the anchor (10) when the patch member (20) is arranged in the groove (11) of the anchor (10) and the anchor (10) is inserted into the borehole (52), wherein said second end section (22) forms a fastening region (26) for connecting the second end section (22) to the tendon or ligament (60).

5. Implant system (1) according to claim 3, **characterized in that** the first end section (21) comprises a width (W) that is greater than a width (W) of the second end section (22).

6. Implant system (1) according to one of the claims 3 to 5, **characterized in that** the first end section (21) and the second end section (22) of the patch member (20) are connected to each other via a middle section (23) of the patch member (20), wherein the middle section (23) is configured to be arranged in said groove (11) of the anchor (10), and wherein the middle section (23) comprises a width (W) that is smaller than a width (W) of the first end section (21) and the second end section (22).

7. Implant system (1) according to one of the preceding claims, **characterized in that** said implant system (1) further comprises an insert (30), which is configured to be arranged in a recess (15) of the anchor (10), such that the insert (30) is configured to contact said bone (50), particularly an inside wall (53) of said borehole (52), when the anchor (10) is inserted into said borehole (52).

8. Implant system (1) according to one of the preceding claims, **characterized in that** said anchor (10) comprises a first side (12), a second side (13) opposite the first side (12), and a bottom side (18) connecting the first side (12) and the second side (13), wherein said groove (11) extends along said first side (12), said bottom side (18) and said second side (13).

9. Implant system (1) according to claim 8, **characterized in that** said anchor comprises a top side (19) connecting said first side (12) and said second side (13), wherein said recess (15) is open towards said first side (12), said second side (13) and said top side (19).

10. Implant system (1) according to claim 8, **characterized in that** said anchor (10) comprises a third side (16) connecting the first side (12) and the second side (13) and a fourth side (17) opposite the third side (16), wherein said recess (15) is open towards said third side (16), said fourth side (17) and said top side (19).

11. Implant system (1) according to one of the preceding claims, **characterized in that** said anchor (10) comprises or consists of an osteoinductive or osteoconductive material.

12. Implant system (1) according to one of the claims 7 to 11, **characterized in that** said insert (30) comprises or consists of an osteoinductive or osteoconductive material.

13. Implant system (1) according to claim 11 or 12, **characterized in that** said osteoinductive or osteoconductive material is selected from the group consisting of tricalcium phosphate, hydroxyapatite, calcium phosphate, calcium silicate and/or human or animal bone.

14. Implant system (1) according to one of the preceding claims, **characterized in that** said patch member (20) comprises a first layer (24) comprising a biocompatible material, particularly collagen, and a second layer (25) connected to the first layer (24), wherein the second layer (25)
- comprises a tear strength of at least 200 N when fully hydrated and/or
- comprises an axial stiffness of at least 20 N/mm when fully hydrated, and/or
- comprises a longitudinal bending stiffness of at least 0,0005 Nm and a lateral bending stiffness of at least 0,005 Nm when fully hydrated, wherein particularly the second layer comprises at least one reinforcing structure for increasing the lateral bending stiffness of the second layer, and/or
- comprises and/or is configured to release a biologically active compound, a drug and/or a cross-linking agent or a collagen cross-linking agent, particularly genipin, wherein the biologically active compound, the drug and/or the cross-linking agent is adapted to diffuse into tissue surrounding the patch member (20) when the patch member (20) is arranged in the groove (11) of the anchor (10) and the anchor (10) is inserted into the borehole (52), wherein particularly the biologically active compound is platelet rich plasma, a natural or recombinant growth factor, a small molecule peptide which is adapted to inhibit or stimulate a resident cell behavior, an anti-inflammatory compound, or a small molecule pharmaceutical agent that is adapted to stimulate mineralized and/or non-mineralized collagen production by cells.

15. Implant system (1) according to one of the preceding claims, **characterized in that** said implant system (1) comprises at least one medial anchor (40) for connecting said patch member (20) to said bone (50), wherein said at least one medial anchor (40) is configured to be inserted into said bone (50)
